(19) Europäisches Patentamt
European Patent Office
Office européen des brevets

(11) Publication number: 0 100 190 B1

(12) **EUROPEAN PATENT SPECIFICATION**

(45) Date of publication of patent specification: 05.10.88

(51) Int. Cl.$^4$: C 07 C 125/065, C 07 C 155/02, A 01 N 47/20

(21) Application number: 83304163.5

(22) Date of filing: 18.07.83

(54) **Fungicidal N-phenylcarbamate.**

(30) Priority: 27.07.82 GB 8221703
06.10.82 GB 8228518

(43) Date of publication of application: 08.02.84 Bulletin 84/06

(45) Publication of the grant of the patent: 05.10.88 Bulletin 88/40

(84) Designated Contracting States: BE CH DE FR GB IT LI NL

(56) References cited:
EP-A-0 051 871
US-A-3 567 425

**The file contains technical information submitted after the application was filed and not included in this specification**

(73) Proprietor: SUMITOMO CHEMICAL COMPANY, LIMITED
15 Kitahama 5-chome Higashi-ku
Osaka-shi Osaka 541 (JP)

(72) Inventor: Noguchi, Hiroshi
10-3-318, Sonehigashi-machi 2-chome
Toyonaka Osaka (JP)
Inventor: Takahashi, Junya
2-303, Ryodo-cho 4-chome
Nishinomiya Hyogo (JP)
Inventor: Yamamoto, Shiego
2-16, Koda 2-chome
Ikeda Osaka (JP)
Inventor: Kato, Toshiro
1-8, D-410, Sakasedai
Takarazuka Hyogo (JP)
Inventor: Oguri, Yukio
10-3-349, Sonehigashi-machi 2-chome
Toyonaka Osaka (JP)
Inventor: Hirata, Naonori
704-2, 7-510, Kanaoka-cho
Sakai Osaka (JP)

(74) Representative: Allard, Susan Joyce et al
BOULT, WADE & TENNANT 27 Furnival Street
London EC4A 1PQ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 100 190 B1

Courier Press, Leamington Spa, England.

**Description**

This invention relates to fungicidal N-phenylcarbamates.

Benzimidazole and thiophanate fungicides such as Benomyl (methyl 1-(butylcarbamoyl)benzimidazol-2-ylcarbamate), Fubelidazol (2-(2-furyl)benzimidazole), Thiabendazole (2-(4-thiazolyl)benzimidazole), Carbendazim (methyl benzimidazol-2-ylcarbamate), Thiophanate-methyl (1,2-bis(3-methoxycarbonyl-2-thioureido)benzene), Thiophanate (1,2-bis(3-ethoxycarbonyl-2-thioureido)benzene), 2-(O,S-dimethylphosphorylamino)-1-(3'-methoxycarbonyl-2'-thioureido)benzene and 2-(O,O-dimethylthiophosphorylamino)-1-(3'-methoxycarbonyl-2'-thioureido)benzene are known to show an excellent fungicidal activity against various plant pathogenic fungi, and they have been widely used as agricultural fungicides since 1970. However, their continuous application over a long period of time results in phytopathogenic fungi developing a tolerance to them, whereby their plant disease-preventive effect is lowered. Furthermore, the fungi which gain a tolerance to certain benzimidazole or thiophanate fungicides also show a considerable tolerance to certain other benzimidazole or thiophanate fungicides. Thus, they are apt to obtain a cross-tolerance. Therefore, if any significant decrease in their plant disease-preventive effect in certain fields is observed, their application in such fields has to be discontinued. However, it is often observed that the density of drug-resistant organisms does not decrease even long after the application is discontinued. Although other fungicides have to be employed in this case, only a few are as effective as benzimidazole or thiophanate fungicides in controlling various phytopathogenic fungi. Cyclic imide fungicides such as Procymidone (3-(3',5'-dichlorophenyl)-1,2-dimethylcyclopropane-1,2-dicarboximide), Iprodione (3-(3',5'-dichlorophenyl)-1-isopropylcarbamoylimidazolidine-2,4-dione), Vinchlozolin (3-(3',5'-(dichlorophenyl)-5-methyl-5-vinyloxazolidine-2,4-dione), ethyl (RS)-3-(3',5'-dichlorophenyl)-5-methyl-2,4-dioxooxazolidine-5-carboxylate, etc., which are effective against various plant diseases, particularly those caused by *Botrytis cinerea,* have the same defects as previously mentioned with respect to the benzimidazole thiophanate fungicides.

C.R. Acad. Sc. Paris, T. 289, S'erie D, pages 691—693 (1979), discloses that herbicides such as Barban (4-chloro-2-butynyl N-(3-chlorophenyl)carbamate), Chlorobufam (1-methyl-2-propynyl N-(3-chlorophenyl)-carbamate), Chloropropham (isopropyl N-(3-chlorophenyl)carbamate) and Propham (isopropyl N-phenylcarbamate) exhibit a fungicidal activity against certain organisms tolerant to some benzimidazole and thiophanate fungicides. However, their fungicidal activity against drug-resistant fungi is not strong enough, and hence in practice they can not be used as fungicides.

We have now found that N-phenylcarbamates of the formula:

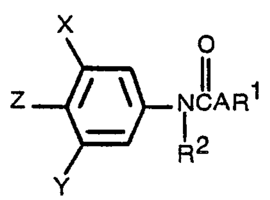

wherein

X is a chlorine atom, a methyl group or an ethynyl group;

Y is a methyl group, a $C_1$—$C_2$ alkoxy group or a methoxymethyl group;

Z is a $C_1$—$C_2$ alkoxy group, a $C_3$ alkenyloxy group, a $C_3$ alkynyloxy group or a halo ($C_1$—$C_2$)alkoxy group;

A is an oxygen atom or a sulfur atom;

$R^1$ is a $C_2$—$C_3$ alkyl group, a methoxy($C_3$)alkyl group or a halo($C_3$)alkyl group;

$R^2$ is a cyano group, a $C_1$—$C_2$ alkyl group, a $C_3$ alkenyl group, $C_3$ alkynyl group, a $C_1$—$C_2$ alkoxycarbonyl-($C_1$—$C_2$)alkyl group or a cyclo($C_3$)alkylmethyl group, or a group of the formula:

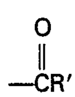

(in which R' is a $C_1$—$C_2$ alkyl group, a cyclo($C_3$)alkyl group, a phenoxymethyl group, a phenyl group or a phenylmethyl group), with the provisos that

when X is a chlorine atom, Y is not a methyl group;

when X is a chlorine atom, Y is $OC_2H_5$ and Z is $—OCH_2CH{=}CH_2$, A is not a sulfur atom;

when X is a chlorine atom and Y is a methoxymethyl group, $R^1$ is not a methoxy($C_3$)alkyl group;

when X is a chlorine atom and Y is a methoxymethyl group, $R^2$ is not a $C_1$—$C_2$ alkoxycarbonylmethyl group; and

when X is an ethynyl group and Y is a $C_1$—$C_2$ alkoxy group, $R^2$ is not a $C_1$—$C_3$ alkyl group.

show an excellent fungicidal activity against plant pathogenic fungi which have developed a resistance to benzimidazole, thiophanate and/or cyclic imide fungicides. It is to be noted that their fungicidal potency

against organisms tolerant to benzimidazole, thiophanate and/or cyclic imide fungicides (hereinafter referred to as "drug-resistant fungi" or "drug-resistant strains") is much higher than that against organisms sensitive to benzimidazole, thiophanate and/or cyclic imide fungicides (hereinafter referred to as "drug-sensitive fungi" or "drug-sensitive strains").

Typical examples of the N-phenylcarbamates of the formula (I) are given in Table 1.

Table 1

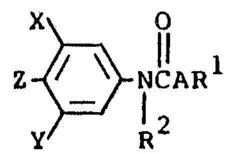

(I)

| X | Y | Z | A | $R^1$ | $R^2$ |
|---|---|---|---|---|---|
| $-CH_3$ | $-CH_3$ | $-OC_2H_5$ | O | $-C_3H_7$(iso) | $-COCH_3$ |
| $-CH_3$ | $-CH_3$ | $-OC_2H_5$ | O | $-C_3H_7$(iso) | -CO- 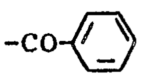 |
| $-CH_3$ | $-CH_3$ | $-OC_2H_5$ | O | $-CH_2CH_2F$ | $-COC_2H_5$ |
| $-CH_3$ | $-CH_3$ | $-OC_2H_5$ | O | 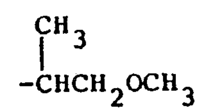 | $-COCH_3$ |
| $-CH_3$ | $-C_3H_7$(n) | $-OC_2H_5$ | O | $-C_3H_7$(iso) | $-COCH_3$ |
| -Cl | $-OCH_3$ | $-OC_2H_5$ | O | $-C_3H_7$(iso) | $-COCH_3$ |
| -Cl | $-OCH_3$ | $-OCH_2C\equiv CH$ | O | $-CH_3$ | $-COCH_3$ |
| -Cl | $-OC_2H_5$ | $-OC_2H_5$ | O | $-C_3H_7$(iso) | $-COCH_3$ |

| X | Y | Z | A | $R^1$ | $R^2$ |
|---|---|---|---|---|---|
| -Cl | $-OC_2H_5$ | $-OCH_2CH=CH_2$ | O | $CH_3$<br>$-CHCH_2OCH_3$ | $-COC_2H_5$ |
| -Cl | $-OC_2H_5$ | $-OCH_2CH_2Cl$ | O | $-C_3H_7$ (iso) | -CO- 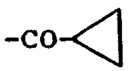 |
| $-CH_3$ | $-OC_2H_5$ | $-OC_2H_5$ | O | $-CH_3$ | -CO- 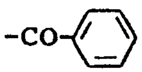 |
| $-CH_3$ | $-OC_2H_5$ | $-OC_2H_5$ | O | $-C_3H_7$ (iso) | $-COCH_3$ |
| $-CH_3$ | $-OC_2H_5$ | $-OC_2H_5$ | O | $-C_3H_7$ (iso) | $-COC_2H_5$ |
| $-CH_3$ | $-OC_2H_5$ | $-OC_2H_5$ | O | $-C_3H_7$ (iso) | -CO- 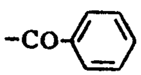 |
| $-CH_3$ | $-OC_2H_5$ | $-OC_2H_5$ | O | $-C_3H_7$ (iso) | -CO- 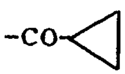 |
| $-CH_3$ | $-OC_2H_5$ | $-OC_2H_5$ | O | $CH_3$<br>$-CHCH_2OCH_3$ | $-COCH_3$ |
| $-CH_3$ | $-OC_2H_5$ | $-OC_2H_5$ | O | $CH_3$<br>$-CHCH_2OCH_3$ | -CO- 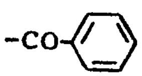 |
| -Cl | $-CH_2OCH_3$ | $-OC_2H_5$ | O | $-C_3H_7$ (iso) | $-COCH_3$ |

| X | Y | Z | A | $R^1$ | $R^2$ |
|---|---|---|---|---|---|
| $-Cl$ | $-CH_2OCH_3$ | $-OC_2H_5$ | O | $CH_3$<br>$-CHCH_2OCH_3$ | $-COCH_3$ |
| $-Cl$ | $-CH_2OCH_3$ | $-OC_2H_5$ | O | $-C_3H_7$ (iso) | -CO- 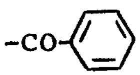 |
| $-Cl$ | $-CH_2OCH_3$ | $-OC_2H_5$ | O | $-C_3H_7$ (iso) | $-COCH_2O-$ 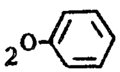 |
| $-Cl$ | $-CH_2OCH_3$ | $-OC_2H_5$ | O | $-C_3H_7$ (iso) | -CO- 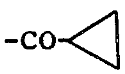 |
| $-Cl$ | $-CH_2OCH_3$ | $-OCH_2C{\equiv}CH$ | O | $-C_3H_7$ (iso) | -CO- 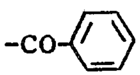 |
| $-CH_3$ | $-CH_2OCH_3$ | $-OC_2H_5$ | O | $-C_3H_7$ (iso) | $-COCH_3$ |
| $-CH_3$ | $-CH_2OCH_3$ | $-OC_2H_5$ | O | $-C_3H_7$ (iso) | -CO- 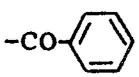 |
| $-CH_3$ | $-CH_2OCH_3$ | $-OC_2H_5$ | O | $CH_3$<br>$-CHCH_2OCH_3$ | $-COCH_3$ |
| $-CH_3$ | $-CH_2OCH_3$ | $-OC_2H_5$ | O | $CH_3$<br>$-CHCH_2OCH_3$ | -CO- 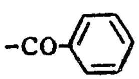 |

| X | Y | Z | A | $R^1$ | $R^2$ |
|---|---|---|---|---|---|
| $-C{\equiv}CH$ | $-OC_2H_5$ | $-OC_2H_5$ | O | $-C_3H_7$ (iso) | -CO-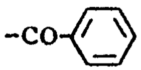 |
| -Cl | $-OC_2H_5$ | $-OC_2H_5$ | S | $-C_3H_7$ (iso) | $-COCH_3$ |
| $-CH_3$ | $-OC_2H_5$ | $-OC_2H_5$ | S | $-C_3H_7$ (iso) | -CO-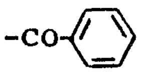 |
| -Cl | $-CH_2OCH_3$ | $-OC_2H_5$ | S | $-C_2H_5$ | -CO-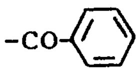 |
| $-CH_3$ | $-CH_3$ | $-OC_2H_5$ | O | $-C_3H_7$ (iso) | $-CH_3$ |
| $-CH_3$ | $-CH_3$ | $-OC_2H_5$ | O | $-C_3H_7$ (iso) | $-CH_2CO_2C_2H_5$ |
| $-CH_3$ | $-CH_3$ | $-OC_2H_5$ | O | $-C_3H_7$ (iso) | 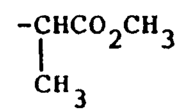 |
| $-CH_3$ | $-CH_3$ | $-OC_2H_5$ | O | $-CH_2CH_2F$ | 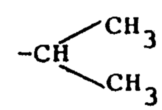 |
| $-CH_3$ | $-C_3H_7$ (n) | $-OC_2H_5$ | O | $-C_3H_7$ (iso) | $-CH_3$ |
| -Cl | $-OCH_3$ | $-OC_2H_5$ | O | $-C_3H_7$ (iso) | $-C_3H_7$ (n) |
| -Cl | $-OC_2H_5$ | $-OC_2H_5$ | O | $-C_3H_7$ (iso) | $-CH_3$ |

| X | Y | Z | A | $R^1$ | $R^2$ |
|---|---|---|---|---|---|
| -Cl | $-OC_2H_5$ | $-OCH_2CH=CH_2$ | O | $CH_3$<br>$-CHCH_2OCH_3$ | $-C_2H_5$ |
| $-CH_3$ | $-OC_2H_5$ | $-OC_2H_5$ | O | $-C_3H_7$(iso) | $-CH_3$ |
| $-CH_3$ | $-OC_2H_5$ | $-OC_2H_5$ | O | $-C_3H_7$(iso) | $-CH_2CO_2C_2H_5$ |
| $-CH_3$ | $-OC_2H_5$ | $-OC_2H_5$ | O | $-C_3H_7$(iso) | $-CH=CHCH_3$ |
| $-CH_3$ | $-OC_2H_5$ | $-OC_2H_5$ | O | $-C_3H_7$(iso) | $-CH_2COC_2H_5$ |
| $-CH_3$ | $-OC_2H_5$ | $-OC_2H_5$ | O | $-C_3H_7$(iso) | $-CH_2C\equiv CH$ |
| $-CH_3$ | $-OC_2H_5$ | $-OC_2H_5$ | O | $CH_3$<br>$-CHCH_2OCH_3$ | $-CH_2-$ 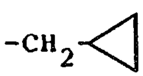 |
| $-CH_3$ | $-OC_2H_5$ | $-OC_2H_5$ | O | $CH_3$<br>$-CHCH_2OCH_3$ | $-CH_2CH=CH_2$ |
| -Cl | $-CH_2OCH_3$ | $-OC_2H_5$ | O | $-C_3H_7$(iso) | $-CH_3$ |
| -Cl | $-CH_2OCH_3$ | $-OC_2H_5$ | O | $-C_3H_7$(iso) | -CN |

| X | Y | Z | A | $R^1$ | $R^2$ |
|---|---|---|---|---|---|
| -Cl | $-CH_2OCH_3$ | $-OC_2H_5$ | O | $-C_3H_7$ (iso) | $CH_3$<br>$-CHCO_2CH_3$ |
| $-CH_3$ | $-CH_2OCH_3$ | $-OC_2H_5$ | O | $-C_3H_7$ (iso) | $-CH_3$ |
| $-CH_3$ | $-CH_2OCH_3$ | $-OC_2H_5$ | O | $CH_3$<br>$-CHCH_2OCH_3$ | $-CH_3$ |
| $-CH_3$ | $-CH_2OCH_3$ | $-OC_2H_5$ | O | $CH_3$<br>$-CHCH_2OCH_3$ | $-CH_2CO_2CH_3$ |
| -Cl | $-OC_2H_5$ | $-OC_2H_5$ | S | $-C_3H_7$ (iso) | $-CH_3$ |
| $-CH_3$ | $-OC_2H_5$ | $-OC_2H_5$ | S | $-C_3H_7$ (iso) | $-CH_2CO_2C_2H_5$ |
| -Cl | $-CH_2OCH_3$ | $-OC_2H_5$ | O | $-C_3H_7$ (iso) | $-COCH_2$- 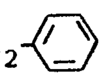 |
| -Cl | $-CH_2OCH_3$ | $-OC_2H_5$ | O | $CH_3$<br>$-CHCH_2OCH_3$ | -CO- 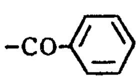 |

Thus, the present invention provides a fungicidal composition which comprises, as an active ingredient, a fungicidally effective amount of the N-phenylcarbamate (I) together with an inert carrier or diluent. It also provides a combination composition comprising as active ingredients the N-phenylcarbamate (I) together with a benzimidazole, thiophanate and/or a cyclic imide fungicide, which is fungicidally effective against not only drug-sensitive fungi but also drug-resistant fungi, and hence particularly effective for the prevention of plant diseases. It also provides a method of controlling plant pathogenic fungi, including drug-resistant strains and drug-sensitive strains, which comprises applying a fungicidally effective amount of the N-phenylcarbamate (I) to plant pathogenic fungi. It further provides novel N-phenylcarbamates which are represented by the formula (I) wherein X, Y, Z, A, $R^1$ and $R^2$ are each as defined above with the proviso that Z is not a hydrogen atom. It furthermore provides a process for producing the novel N-phenylcarbamates (I).

The N-phenylcarbamates (I) can be prepared by reacting an N-phenylcarbamate of the formula:

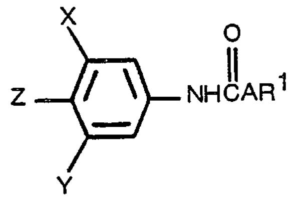

(II)

wherein X, Y, Z, A and $R^1$ are each as defined above, with a halide or an acid anhydride of the formula:

$R^2$—W (III)

or

$(R^8CO)_2O$ (IV)

wherein $R^2$ and $R^8$ are each as defined above and W is halogen; or (b) reacting an aniline derivative of the formula:

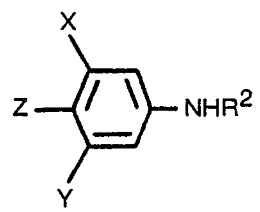

(V)

wherein X, Y, Z and $R^2$ are each as defined above, with a chloroformate of the formula:

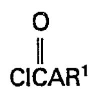

(VI)

wherein A and $R^1$ are each as defined above.

The starting N-phenylcarbamate (II) can be produced by a known process (cf. EP—A—0063905) or any process analogous thereto.

The N-phenylcarbamates (I) are fungicidally effective against a wide variety of plant pathogenic fungi, of which examples are as follows: *Podosphaera leucotricha, Venturia inaequalis, Mycosphaerella pomi, Marssonina mali* and *Sclerotinia mali* of apple, *Phyllactinia kakicola* and *Gloeosporium kaki* or persimmon, *Cladosporium carpophilum* and *Phomopsis* sp. of peach, *Cercospora viticola, Uncinula necator, Elsinoe ampelina* and *Glomerella cingulata* of grape, *Cercospora beticola* of sugarbeet, *Cercospora arachidicola* and *Cercospora personata* of peanut, *Erysiphe graminis* f. sp. *hordei, Cercosporella herpotrichoides* and *Fusarium nivale* of barley, *Erysiphe graminis* f. sp. *tritici* of wheat, *Sphaerotheca fuliginea* and *Cledosporium cucumerinum* of cucumber, *Cladosporium fulvum* of tomato, *Corynespora melongenae* of eggplant, *Sphaerotheca humuli, Fusarium oxysporum* f. sp. *fragariae* of strawberry, *Botrytis alli* of onion, *Cercospora apii* of celery, *Phaeoisariopsis griseola* of kidney bean, *Erysiphe cichoracearum* of tobacco, *Diplocarpon rosae* of rose, *Elsinoe fawcetti, Penicillium italicum, Penicillium digitatum* of orange, *Bostrytis cinerea* of cucumber, eggplant, tomato, strawberry, pimiento, onion, lettuce, grape, orange, cyclamen, rose or hop, *Sclerotinia sclerotiorum* of cucumber, eggplant, pimiento, lettuce, celery, kidney bean, soybean, azuki bean, potato or sunflower, *Sclerotinia cinerea* of peach or cherry, *Mycosphaerella melonis* of cucumber or melon, etc. Namely, the N-phenylcarbamates (I) are highly effective in controlling the drug-resistant strains of the fungi.

The N-phenylcarbamates (I) are also fungicidally effective against fungi sensitive to said known fungicides as well as fungi against which said known fungicides are ineffective. Examples of such fungi are *Pyricularia oryzae, Pseudoperonospora cubensis, Plasmopara viticola, Phytophthora infestans,* etc.

Advantageously, the N-phenylcarbamates (I) are low in toxicity and hence have little detrimental effect on mammals etc.

They may also be applied to agricultural fields without causing any material toxicity to important crop plants.

Typical examples of the processes for the preparation of the N-phenylcarbamates (I) are illustratively shown in the following examples.

Example 1

Preparation of isopropyl N-acetyl-N-(3-chloro-4-ethoxy-5-methoxymethylphenyl)carbamate according to Procedure (a):—

Isopropyl N-(3-chloro-4-ethoxy-5-methoxymethylphenyl)carbamate (3.02 g) was dissolved in acetic anhydride (50 ml), and two drops of conc. sulfuric acid were added thereto. The mixture was refluxed for 30 minutes, poured into ice-water and extracted with ether. The extract was washed with sodium bicarbonate solution and saturated brine, dried over anhydrous magnesium sulfate and concentrated under reduced pressure. The residue was purified by silica gel chromatography using hexane-acetone as the eluent to give isopropyl N-acetyl-N-(3-chloro-4-ethoxy-5-methoxymethylphenyl)carbamate (Compound No. 22) (2.71 g) in a yield of 67%. $n_D^{26}$ 1.4954.

Example 2

Preparation of isopropyl N-benzoyl-N-(3,4-diethoxy-5-methylphenyl)carbamate according to Procedure (a):—

Isopropyl N-(3,4-diethoxy-5-methylphenyl)carbamate (2.81 g) was dissolved in dimethylformamide (50 ml), and sodium hydride dispersion (50%, 0.5 g) was added thereto. The mixture was heated at 60°C for 15 minutes, treated with benzoyl chloride (1.41 g) and heated for 30 minutes. The reaction mixture was poured into ice-water and extracted with ether. The extract was washed with sodium bicarbonate solution and saturated brine, dried over anhydrous magnesium sulfate and concentrated under reduced pressure. The residue was purified by silica gel chromatography using hexane-acetone as the eluent to give isopropyl N-benzoyl-N-(3,4-diethoxy-5-methylphenyl)carbamate (Compound No. 20) (3.35 g) in a yield of 87%. $n_D^{26}$ 1.5313.

Example 3

Preparation of isopropyl N-benzoyl-N-(3-chloro-4-ethoxy-5-methoxymethylphenyl)carbamate according to Procedure (b):—

N-(3-Chloro-4-ethoxy-5-methoxymethylbenzamide (3.2 g) was dissolved in dimethylformamide (50 ml), and sodium hydride dispersion (50%, 0.5 g) was added thereto. The mixture was heated at 60°C for 15 minutes, treated with isopropyl chloroformate (1.23 g) and heated for 30 minutes. The reaction mixture was poured into ice-water and extracted with ether. The extract was washed with sodium bicarbonate solution and saturated brine, dried over anhydrous magnesium sulfate and concentrated under reduced pressure. The residue was purified by silica gel chromatography using hexane-acetone as the eluent to give isopropyl N-benzoyl-N-(3-chloro-4-ethoxy-5-methoxymethylphenyl)carbamate (Compound No. 23) (3.37 g) in a yield of 83%. $n_D^{26}$ 1.5307.

Example 4

Preparation of isopropyl N-methyl-N-(3-chloro-4-ethoxy-5-methoxymethylphenyl)carbamate according to Procedure (a):—

To a mixture of isopropyl N-(3-chloro-4-ethoxy-5-methoxymethylphenyl)carbamate (2.0 g), tetrahydrofuran (30 ml), powdered potassium hydroxide (0.56 g) and tetra-n-butylammonium bromide (0.5 g) was added methyl iodide (1.88 g) with stirring at 25°C. After 1.5 hours, the reaction mixture was refluxed for 3 hours, then cooled to room temeprature, poured into water and extracted with toluene. The extract was washed with diluted hydrochloride and water, dried over anhydrous magnesium sulfate and concentrated under reduced pressure. The residue was purified by silica gel chromatography using toluene-tetrahydrofuran as the eluent to give isopropyl N-methyl-N-(3-chloro-4-ethoxy-5-methoxymethylphenyl)carbamate (Compound No. 41) (2.01 g) in a yield of 96%. $n_D^{25}$ 1.5104.

Example 5

Preparation of isopropyl N-ethoxycarbonylmethyl-N-(3,4-diethoxy-5-methylphenyl)carbamate according to procedure (a):—

To a mixture of isopropyl N-(3,4-diethoxy-5-methylphenyl)carbamate (2.0 g), tetrahydrofuran (30 ml), powdered potassium hydroxide (0.60 g) and tetra-n-butylammonium bromide (0.5 g) was added ethyl 2-bromoacetate (2.38 g) with stirring at 25°C. After 2 hours, the reaction mixture was refluxed for 5 hours, then cooled to room temperature, poured into water and extracted with toluene. The extract was washed with diluted hydrochloride and water, dried over anhydrous magnesium sulfate and concentrated under reduced pressure. The residue was purified by silica gel chromatography using toluene-tetrahydrofuran as the eluent to give isopropyl N-ethoxycarbonylmethyl-N-(3,4-diethoxy-5-methylphenyl)carbamate (Compound No. 36) (2.40 g) in a yield of 92%. $n_D^{26}$ 1.4921.

Example 6

Preparation of isopropyl N-propargyl-N-(3,4-diethoxy-5-methylphenyl)carbamate according to Procedure (b):—

To a solution of N-propargyl-N-(3,4-diethoxy-5-methyl)aniline (2.0 g) in 30 ml of toluene were added isopropyl chloroformate (1.37 g) and N,N-diethylaniline (1.66 g) with stirring at 25°C. After 2 hours, the reaction mixture was refluxed for 2 hours, then cooled to room temperature, poured into water and extracted with toluene. The extract was washed with diluted hydrochloride and water, dried over anhydrous magnesium sulfate and concentrated under reduced pressure. The residue was purified by silica gel chromatography using toluene-tetrahydrofuran as the eluent to give isopropy N-propargyl-N-(3,4-diethoxy-5-methylphenyl)carbamate (Compound No. 38) (2.63 g) in a yield of 96.0%. $n_D^{26}$ 1.5032.

According to the above procedures, the N-phenylcarbamate of the formula (I) as shown in Table 2 may be prepared.

Table 2

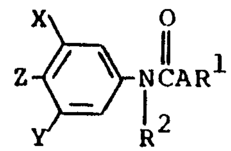

(I)

| Compound No. | X | Y | Z | A | $R^1$ | $R^2$ | Physical constant |
|---|---|---|---|---|---|---|---|
| 9 | $-CH_3$ | $-CH_3$ | $-OC_2H_5$ | O | $-C_3H_7$(iso) | $-COCH_3$ | M.P. 62-63°C |
| 10 | $-CH_3$ | $-CH_3$ | $-OC_2H_5$ | O | $-C_3H_7$(iso) | -CO- 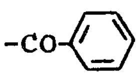 | $n_D^{20.5}$ 1.5299 |
| 11 | $-CH_3$ | $-CH_3$ | $-OC_2H_5$ | O | $-CH_2CH_2F$ | $-COC_2H_5$ | M.P. 26-27°C |
| 12 | $-CH_3$ | $-CH_3$ | $-OC_2H_5$ | O | $CH_3$<br>$-CHCH_2OCH_3$ | $-COCH_3$ | $n_D^{22.5}$ 1.4922 |
| 13 | -Cl | $-OCH_3$ | $-OC_2H_5$ | O | $-C_3H_7$(iso) | $-COCH_3$ | $n_D^{24}$ 1.5113 |
| 14 | -Cl | $-OC_2H_5$ | $-OC_2H_5$ | O | $-C_3H_7$(iso) | $-COCH_3$ | $n_D^{21}$ 1.5096 |
| 16 | -Cl | $-OC_2H_5$ | $-OCH_2CH=CH_2$ | O | $CH_3$<br>$-CHCH_2OCH_3$ | $-COC_2H_5$ | $n_D^{20.5}$ 1.5109 |
| 17 | -Cl | $-OC_2H_5$ | $-OCH_2CH_2Cl$ | O | $-C_3H_7$(iso) | -CO- 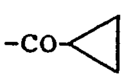 | M.P. 54-56.5°C |

| Compound No. | X | Y | Z | A | $R^1$ | $R^2$ | Physical constant |
|---|---|---|---|---|---|---|---|
| 18 | $-CH_3$ | $-OC_2H_5$ | $-OC_2H_5$ | O | $-C_3H_7$(iso) | $-COCH_3$ | $n_D^{24}$ 1.4961 |
| 19 | $-CH_3$ | $-OC_2H_5$ | $-OC_2H_5$ | O | $-C_3H_7$(iso) | $-COC_2H_5$ | $n_D^{26}$ 1.4951 |
| 20 | $-CH_3$ | $-OC_2H_5$ | $-OC_2H_5$ | O | $-C_3H_7$(iso) | -CO- 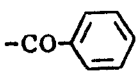 | $n_D^{26}$ 1.5313 |
| 21 | $-CH_3$ | $-OC_2H_5$ | $-OC_2H_5$ | O | $CH_3$<br>$-CHCH_2OCH_3$ | $-COCH_3$ | $n_D^{26.5}$ 1.5000 |
| 22 | -Cl | $-CH_2OCH_3$ | $-OC_2H_5$ | O | $-C_3H_7$(iso) | $-COCH_3$ | $n_D^{26}$ 1.4954 |
| 23 | -Cl | $-CH_2OCH_3$ | $-OC_2H_5$ | O | $-C_3H_7$(iso) | -CO- 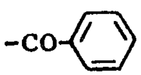 | $n_D^{26}$ 1.5307 |
| 24 | -Cl | $-CH_2OCH_3$ | $-OC_2H_5$ | O | $-C_3H_7$(iso) | $-COCH_2O$- 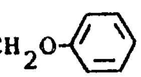 | $n_D^{21}$ 1.5353 |
| 25 | -Cl | $-CH_2OCH_3$ | $-OC_2H_5$ | O | $-C_3H_7$(iso) | -CO- 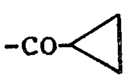 | $n_D^{23}$ 1.5129 |

| Compound No. | X | Y | Z | A | $R^1$ | $R^2$ | Physical constant |
|---|---|---|---|---|---|---|---|
| 26 | -Cl | $-CH_2OCH_3$ | $-OCH_2C{\equiv}CH$ | O | $-C_3H_7$(iso) | -CO- 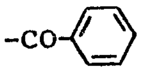 | $n_D^{27}$ 1.5494 |
| 27 | $-C{\equiv}CH$ | $-OC_2H_5$ | $-OC_2H_5$ | O | $-C_3H_7$(iso) | -CO- 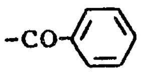 | $n_D^{27.5}$ 1.5387 |
| 28 | -Cl | $-CH_2OCH_3$ | $-OC_2H_5$ | S | $-C_2H_5$ | -CO- 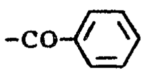 | $n_D^{26}$ 1.5778 |
| 31 | $-CH_3$ | $-CH_3$ | $-OC_2H_5$ | O | $-C_3H_7$(iso) | $-CH_3$ | $n_D^{27}$ 1.5023 |
| 32 | $-CH_3$ | $-CH_3$ | $-OC_2H_5$ | O | $-C_3H_7$(iso) | $-CH_2CO_2C_2H_5$ | $n_D^{29}$ 1.4871 |
| 33 | -Cl | $-OCH_3$ | $-OC_2H_5$ | O | $-C_3H_7$(iso) | $-C_3H_7$(n) | $n_D^{27}$ 1.5061 |
| 35 | $-CH_3$ | $-OC_2H_5$ | $-OC_2H_5$ | O | $-C_3H_7$(iso) | $-CH_3$ | $n_D^{27}$ 1.4991 |
| 36 | $-CH_3$ | $-OC_2H_5$ | $-OC_2H_5$ | O | $-C_3H_7$(iso) | $-CH_2CO_2C_2H_5$ | $n_D^{26}$ 1.4921 |
| 38 | $-CH_3$ | $-OC_2H_5$ | $-OC_2H_5$ | O | $-C_3H_7$(iso) | $-CH_2C{\equiv}CH$ | $n_D^{26}$ 1.5032 |

| Compound No. | X | Y | Z | A | $R^1$ | $R^2$ | Physical constant |
|---|---|---|---|---|---|---|---|
| 39 | $-CH_3$ | $-OC_2H_5$ | $-OC_2H_5$ | O | $CH_3$<br>\|<br>$-CHCH_2OCH_3$ | $-CH_2-$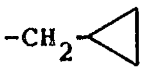 | $n_D^{25}$ 1.5042 |
| 40 | $-CH_3$ | $-OC_2H_5$ | $-OC_2H_5$ | O | $CH_3$<br>\|<br>$-CHCH_2OCH_3$ | $-CH_2CH=CH_2$ | $n_D^{27}$ 1.5027 |
| 41 | -Cl | $-CH_2OCH_3$ | $-OC_2H_5$ | O | $-C_3H_7$(iso) | $-CH_3$ | $n_D^{25}$ 1.5104 |
| 43 | -Cl | $-CH_2OCH_3$ | $-OC_2H_5$ | O | $-C_3H_7$(iso) | -CN | $n_D^{23.5}$ 1.5102 |
| 45 | -Cl | $-CH_2OCH_3$ | $-OC_2H_5$ | O | $-C_3H_7$(iso) | $CH_3$<br>\|<br>$-CHCO_2CH_3$ | $n_D^{25}$ 1.5078 |
| 50 | -Cl | $-CH_2OCH_3$ | $-OC_2H_5$ | O | $-C_3H_7$(iso) | $-COCH_2-$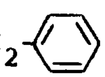 | $n_D^{21}$ 1.5346 |
| 52 | -Cl | $-CH_2OCH_3$ | $-OC_2H_5$ | O | $CH_3$<br>\|<br>$-CHCH_2OCH_3$ | -CO-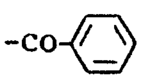 | $n_D^{20}$ 1.5410 |

In the practical usage of the N-phenylcarbamates (I) as a fungicide, they may be applied as such or in a formulation form such as dusts, wettable powders, oil sprays, emulsifiable concentrates, tablets, granules, fine granules, aerosols or flowables. Such formulation form can be formulated in a conventional manner by mixing at least one of the N-phenylcarbamates (I) with an appropriate solid or liquid carrier(s) or diluent(s) and, if necessary, an appropriate adjuvant(s) (e.g. surfactants, adherents, dispersants, stabilizers) for improving the dispersibility and other properties of the active ingredient.

Examples of the solid carriers or diluents are botanical materials (e.g. flour, tobacco stalk powder, soybean powder, walnut-shell powder, vegetable powder, saw dust, bran, bark powder, cellulose powder, vegetable extract residue), fibrous materials (e.g. paper, corrugated cardboard, old rags), synthetic plastic powders, clays (e.g. kaolin, bentonite, fuller's earth), talcs, other inorganic materials (e.g. pyrophyllite, sericite, pumice, sulfur powder, active carbon) and chemical fertilizers (e.g. ammonium sulfate, ammonium phosphate, ammonium nitrate, urea, ammonium chloride).

Examples of the liquid carriers or diluents are water, alcohols (e.g. methanol, ethanol), ketones (e.g. acetone, methylethylketone), ethers (e.g. diethyl ether, dioxane, cellosolve, tetrahydrofuran), aromatic hydrocarbons (e.g. benzene, toluene, xylene, methyl naphthalene), aliphatic hydrocarbons (e.g. gasoline, kerosene, lamp oil), esters, nitriles, acid amides (e.g. dimethylformamide, dimethylacetamide), halogenated hydrocarbons (e.g. dichloroethane, carbon tetrachloride), etc.

Examples of the surfactants are alkyl sulfuric esters, alkyl sulfonates, alkylaryl sulfonates, polyethylene glycol ethers, polyhydric alcohol esters, etc. Examples of the adherents and dispersants may include cesein, gelatin, starch powder, carboxymethyl cellulose, gum arabic, alginic acid, lignin, bentonite, molasses, polyvinyl alcohol, pine oil and agar. As the stabilizers, there may be used PAP (isopropyl acid phosphate mixture), tricresyl phosphate (TCP), tolu oil, epoxydized oil, various surfactants, various fatty acids and their esters, etc.

The foregoing formulations generally contain at least one of the N-phenylcarbamates (I) in a concentration of about 1 to 95% by weight, preferably of 2.0 to 80% by weight. By using the formulations, the N-phenylcarbamates (I) are generally applied in such amounts as 2 to 100 g per 10 are.

When only the drug-resistant strains of phytopathogenic fungi are present, the N-phenylcarbamates (I) may be used alone. However, when the drug-sensitive strains are present together with the drug-resistant strains, their alternate use with benzimidazole, thiophanate and/or cyclic imide fungicides or their combined use with benzimidazole, thiophanate and/or cyclic imide fungicides is preferred. In such alternate or combined use, each active ingredient may be employed as such or in conventional agricultural formulation forms. For the combined use, the weight proportion of the N-phenylcarbamate (I) and the benzimidazole, thiophanate and/or the cyclic imide fungicide may be from about 1:0.1 to 1:10.0.

Typical examples of the benzimidazole and thiophanate fungicides and the cyclic imide fungicides are given in Table 3.

Table 3

| Compound | Structure | Name |
|---|---|---|
| A | 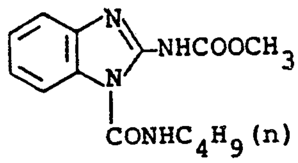 | Methyl 1-(butylcarbamoyl)benzimidazol-2-ylcarbamate |
| B | 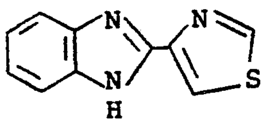 | 2-(4-Thiazolyl)benzimidazole |
| C | 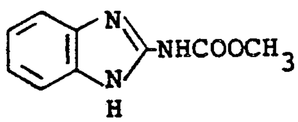 | Methyl benzimidazol-2-ylcarbamate |
| D | 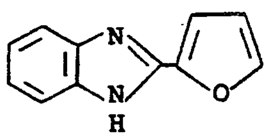 | 2-(2-Furyl)benzimidazole |
| E | 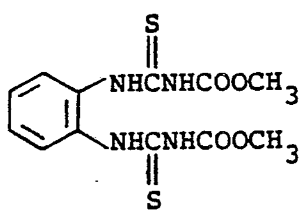 | 1,2-Bis(3-methoxycarbonyl-2-thioureido)benzene |
| F | 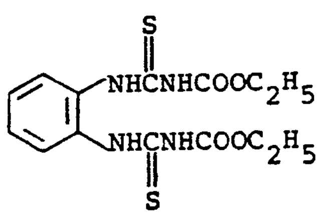 | 1,2-Bis(3-ethoxycarbonyl-2-thioureido)benzene |
| G | 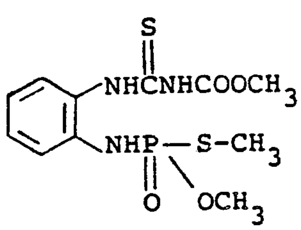 | 2-(O,S-Dimethylphosphorylamino)-1-(3'-methoxycarbonyl-2'-thioureido)benzene |
| H | 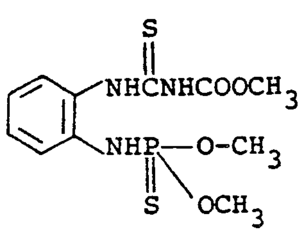 | 2-(O,O-Dimethylthiophosphorylamino)-1-(3'-methoxycarbonyl-2'-thioureido)benzene |

| Compound | Structure | Name |
|---|---|---|
| I | 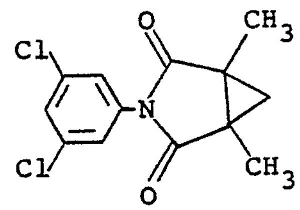 | N-(3',5'-Dichlorophenyl)-1,2-dimethylcyclopropane-1,2-dicarboximide |
| J | 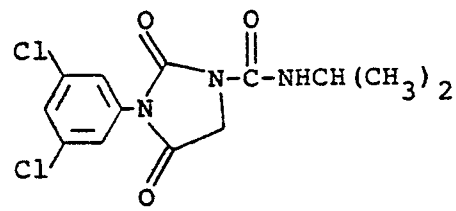 | 3-(3',5'-Dichlorophenyl)-1-isopropylcarbamoylimidazolidine-2,4-dione |
| K | 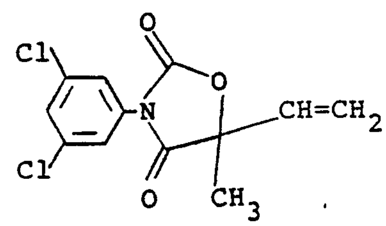 | 3-(3',5'-Dichlorophenyl)-5-methyl-5-vinyloxazolidine-2,4-dione |
| L | 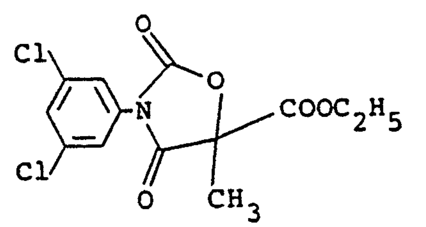 | Ethyl (RS)-3-(3',5'-dichlorophenyl)-5-methyl-2,4-dioxo-oxazolidine-5-carboxylate |

The N-phenylcarbamates (I) may also be used in admixture with other fungicides, herbicides, insecticides, miticides, fertilizers, etc.

When the N-phenylcarbamates (I) are used as fungicides, they may be applied in amounts of from 2 to 100 grams per 10 ares. However, this amount may vary depending upon formulation forms, application times, application methods, application sites, diseases, crops and so on, and therefore, they are not limited to these particular amounts.

Some practical embodiments of the fungicidal composition according to the invention are illustratively shown in the following Examples wherein % and part(s) are by weight.

Formulation Example 1

Two parts of Compound No. 22, 88 parts of clay and 10 parts of talc are thoroughly pulverized and mixed together to obtain a dust formulation containing 2% of the active ingredient.

Formulation Example 2

Fifty parts of Compound No. 20, 25 parts of diatomaceous earth, 20 parts of white carbon, 3 parts of sodium laurylsulfate as a wetting agent and 2 parts of calcium ligninsulfonate as a dispersing agent are mixed while being powdered to obtain a wettable powder formulation containing 50% of the active ingredient.

Formulation Example 3

One part of Compound No. 23, 1 part of Compound I, 88 parts of clay and 10 parts of talc are thoroughly pulverized and mixed together to obtain a dust formulation containing 2% of the active ingredient.

Formulation Example 4

Twenty parts of Compound No. 10, 10 parts of Compound J, 45 parts of diatomaceous earth, 20 parts of white carbon, 3 parts of sodium laurylsulfate as a wetting agent and 2 parts of calcium ligninsulfonate as a dispersing agent are mixed while being powdered to obtain a wettable powder formulation containing 30% of the active ingredient.

Formulation Example 5

Two parts of Compound No. 41, 88 parts of clay and 10 parts of talc are thoroughly pulverized and mixed together to obtain a dust formulation containing 2% of the active ingredient.

Formulation Example 6

Fifty parts of Compound No. 38, 25 parts of diatomaceous earth, 20 parts of white carbon, 3 parts of sodium laurylsulfate as a wetting agent and 2 parts of calcium ligninsulfonate as a dispersing agent are mixed while being powdered to obtain a wettable powder formulation containing 50% of the active ingredient.

Formulation Example 7

One part of Compound No. 45, 1 part of Compound I, 88 parts of clay and 10 parts of talc are thoroughly pulverized and mixed together to obtain a dust formulation containing 2% of the active ingredient.

Typical test data indicating the excellent fungicidal activity of the N-phenylcarbamates (I) are shown below. The compounds used for comparison are as follows:

| Compound | Remarks |
|---|---|
| Swep 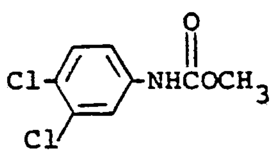 | Commercially available herbicide |
| Chlorpropham 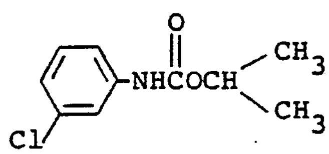 | Commercially available herbicide |
| Barban 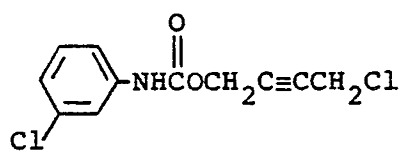 | Commercially available herbicide |
| CEPC 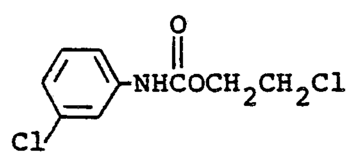 | Commercially available herbicide |
| Propham 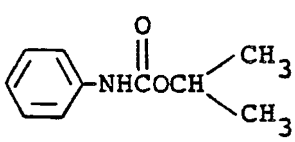 | Commercially available herbicide |

| Compound | Remarks |
|---|---|
| Chlorbufam 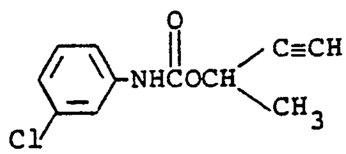 | Commercially available herbicide |
| Benomyl 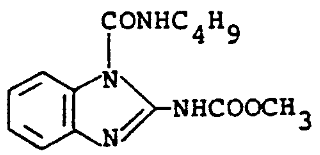 | Commercially available fungicide |
| Thiophanate-methyl 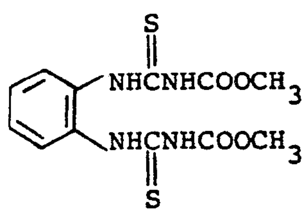 | Commercially available fungicide |
| Carbendazim 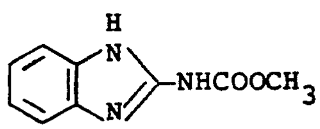 | Commercially available fungicide |
| Thiabendazole 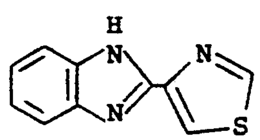 | Commercially available fungicide |
| Edifenphos 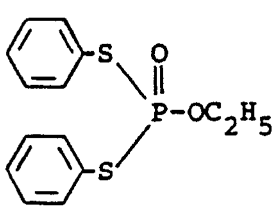 | Commercially available fungicide |

Experiment 1

Protective activity test on powdery mildew of cucumber (*Sphaerotheca fuliginea*):—

A flower pot of 90 ml volume was filled with sandy soil, and seeds of cucumber (var: Sagami-hanjiro) were sowed therein. Cultivation was carried out in a greenhouse for 8 days. Onto the resulting seedlings having cotyledons, the test compound formulated in emulsifiable concentrate or wettable powder and diluted with water was sprayed at a rate of 10 ml per pot. Then, the seedlings were inoculated with a spore suspension of the drug-resistant or drug-sensitive strain of *Sphaerotheca fuliginea* by spraying and further

cultivated in the greenhouse. Ten days thereafter, the infectious state of the plants were observed. The degree of damage was determined in the following manner, and the results are shown in Table 4.

The leaves examined were measured for a percentage of infected area and classified into the corresponding disease indices, 0, 0.5, 1, 2, 4:

| Disease index | Percentage of infected area |
|---|---|
| 0 | No infection |
| 0.5 | Infected area of less than 5% |
| 1 | Infected area of less than 20% |
| 2 | Infected area of less than 50% |
| 4 | Infected area of not less than 50% |

The disease severity was calculated according to the following equation:

$$\text{Disease severity (\%)} = \frac{\Sigma\{(\text{Disease index}) \times (\text{Number of leaves})\}}{4 \times (\text{Total number of leaves examined})} \times 100$$

The prevention value was calculated according to the following equation:

$$\text{Prevention value (\%)} = 100 - \frac{(\text{Disease severity in treated plot})}{(\text{Disease severity in untreated plot})} \times 100$$

Table 4

| Compound No. | Concentration of active ingredient (ppm) | Prevention value when inoculated with drug-resistant strain (%) | Prevention value when inoculated with drug-sensitive strain (%) |
|---|---|---|---|
| 10 | 200 | 94 | 0 |
| 14 | 200 | 100 | 0 |
| 18 | 200 | 97 | 0 |
| 20 | 200 | 100 | 0 |
| 21 | 200 | 100 | 0 |
| 22 | 200 | 100 | 0 |
| 23 | 200 | 100 | 0 |
| 35 | 200 | 100 | 0 |
| 36 | 200 | 100 | 0 |
| 41 | 200 | 100 | 0 |
| 43 | 200 | 100 | 0 |
| 45 | 200 | 100 | 0 |
| Swep | 200 | 0 | 0 |
| Chlorpropham | 200 | 0 | 0 |
| Barban | 200 | 25 | 0 |
| CEPC | 200 | 0 | 0 |
| Propham | 200 | 0 | 0 |
| Chlorbufam | 200 | 0 | 100 |
| Benomyl | 200 | 0 | 100 |
| Thiophanate-methyl | 200 | 0 | 100 |
| Carbendazim | 200 | 0 | 100 |

As can be seen from the results given in Table 4, the N-phenylcarbamates (I) of the invention show an excellent preventive effect on the drug-resistant strain but do not show any preventive effect on the tested drug-sensitive strain. On the contrary, commercially available known fungicides such as Benomyl, Thiophanate-methyl and Carbendazim show a notable controlling effect on the drug-sensitive strain but not on the drug-resistant strain. Other tested compounds structurally similar to the N-phenyl-carbamates (I) do not show any fungicidal activity on the drug-sensitive strain and the drug-resistant strain.

Experiment 2

Preventive effect on cercospora leaf spot of sugarbeet (*Cercospora beticola*):—

A flower pot of 90 ml volume was filled with sandy soil, and seeds of sugarbeet (var: Detroit dark red) were sowed therein. Cultivation was carried out in a greenhouse for 20 days. Onto the resulting seedlings, the test compound formulated in emulsifiable concentrate or wettable powder and diluted with water was sprayed at a rate of 10 ml per pot. Then, the seedlings were inoculated with a spore suspension of the drug-resistant or drug-sensitive strain of *Cercospora beticola* by spraying. The pot was covered with a polyvinyl chloride sheet to make a condition of high humidity, and cultivation was continued in the greenhouse for 10 days. The degree of damage was determined in the same manner as in Experiment 1, and the results are shown in Table 5.

Table 5

| Compound No. | Concentration of active ingredient (ppm) | Prevention value when inoculated with drug-resistant strain (%) | Prevention value when inoculated with drug-sensitive strain (%) |
|---|---|---|---|
| 14 | 200 | 100 | 0 |
| 18 | 200 | 97 | 0 |
| 20 | 200 | 98 | 0 |
| 22 | 200 | 100 | 0 |
| 23 | 200 | 100 | 0 |
| 25 | 200 | 100 | 0 |
| 35 | 200 | 100 | 0 |
| 36 | 200 | 100 | 0 |
| 43 | 200 | 100 | 0 |
| 45 | 200 | 100 | 0 |
| Swep | 200 | 0 | 0 |
| Chlorpropham | 200 | 0 | 0 |
| Barban | 200 | 34 | 0 |
| CEPC | 200 | 0 | 0 |
| Propham | 200 | 0 | 0 |
| Chlorbufam | 200 | 0 | 100 |
| Benomyl | 200 | 0 | 100 |
| Thiophanate-methyl | 200 | 0 | 100 |
| Carbendazim | 200 | 0 | 100 |

As can be seen from the results given in Table 5, the N-phenylcarbamates (I) of the invention show an excellent preventive effect on the drug-resistant strain but do not show any preventive effect on the tested drug-sensitive strain. On the contrary, commercially available known fungicides such as Benomyl and Thiophanate-methyl show a notable controlling effect on the drug-sensitive strain but not on the drug-resistant strain. Other tested compounds structurally similar to the N-phenylcarbamates (I) do not show any fungicidal activity on the drug-sensitive strain and the drug-resistant strain.

Experiment 3

Preventive effect on scab of pear (*Venturia nashicola*):—

A plastic pot of 90 ml volume was filled with sandy soil, and seeds of pear (var: Chojuro) were sowed therein. Cultivation was carried out in a greenhouse for 20 days. Onto the resulting seedlings, the test compound formulated in emulsifiable concentrate or wettable powder and diluted with water was sprayed

at a rate of 10 ml per pot. Then, the seedlings were inoculated with a spore suspension of the drug-resistant or drug-sensitive strain of *Venturia nashicola* by spraying. The resulting plants were placed at 20°C under a condition of high humidity for 3 days and then at 20°C under irradiation with a fluorescent lamp for 20 days. The degree of damage was determined in the same manner as in Experiment 1, and the results are shown in Table 6.

Table 6

| Compound No. | Concentration of active ingredient (ppm) | Prevention value when inoculated with drug-resistant strain (%) | Prevention value when inoculated with drug-sensitive strain (%) |
|---|---|---|---|
| 10 | 200 | 94 | 0 |
| 20 | 200 | 100 | 0 |
| 22 | 200 | 98 | 0 |
| 23 | 200 | 100 | 0 |
| 25 | 200 | 100 | 0 |
| 33 | 200 | 100 | 0 |
| 41 | 200 | 100 | 0 |
| 43 | 200 | 100 | 0 |
| 45 | 200 | 100 | 0 |
| Benomyl | 200 | 0 | 100 |
| Thiophanate-methyl | 200 | 0 | 100 |

As can be seen from the results given in Table 6, the N-phenylcarbamates (I) of the invention show an excellent preventive effect on the drug-resistant strain but do not show any preventive effect on the tested drug-sensitive strain. On the contrary, commercially available known fungicides such as Benomyl and Thiophanate-methyl show a notable controlling effect on the drug-sensitive strain but not on the drug-resistant strain.

Experiment 4

Preventive effect on gray mold of cucumber (*Botrytis cinerea*):—

Plastic pots of 90 ml volume was filled with sandy soil, and seeds of cucumber (var: Sagami-hanjiro) were sowed therein. Cultivation was carried out in a greenhouse for 8 days to obtain cucumber seedlings expanding cotyledons. Onto the resulting seedlings, the test compound formulated in emulsifiable concentrate or wettable powder and diluted with water was sprayed at a rate of 10 ml per pot. After airdrying, the seedlings were inoculated with mycelial disks (5 mm in diameter) of the drug-resistant or drug-sensitive strain of *Botrytis cinerea* by putting them on the leaf surfaces. After the plants were infected by incubating under high humidity at 20°C for 3 days, the rates of disease severity were observed. The degree of damage was determined in the same manner as in Experiment 1, and the results are shown in Table 7.

Table 7

| Compound No. | Concentration of active ingredient (ppm) | Prevention value when inoculated with drug-resistant strain (%) | Prevention value when inoculated with drug-sensitive strain (%) |
|---|---|---|---|
| 9 | 200 | 100 | 0 |
| 10 | 200 | 100 | 0 |
| 11 | 200 | 100 | 0 |
| 12 | 200 | 100 | 0 |
| 13 | 200 | 100 | 0 |
| 14 | 200 | 100 | 0 |
| 16 | 200 | 100 | 0 |
| 17 | 200 | 100 | 0 |
| 18 | 200 | 100 | 0 |
| 19 | 200 | 100 | 0 |
| 20 | 200 | 100 | 0 |
| 21 | 200 | 100 | 0 |
| 22 | 200 | 100 | 0 |
| 23 | 200 | 100 | 0 |
| 24 | 200 | 100 | 0 |
| 25 | 200 | 100 | 0 |
| 26 | 200 | 100 | 0 |
| 27 | 200 | 100 | 0 |
| 28 | 200 | 100 | 0 |
| 31 | 200 | 100 | 0 |
| 32 | 200 | 100 | 0 |
| 33 | 200 | 100 | 0 |
| 35 | 200 | 100 | 0 |
| 36 | 200 | 100 | 0 |
| 38 | 200 | 100 | 0 |
| 39 | 200 | 100 | 0 |
| 40 | 200 | 100 | 0 |
| 41 | 200 | 100 | 0 |
| 43 | 200 | 100 | 0 |
| 45 | 200 | 100 | 0 |
| 50 | 200 | 100 | 0 |
| 52 | 200 | 100 | 0 |
| Benomyl | 200 | 0 | 100 |
| Thiophanate-methyl | 200 | 0 | 100 |

As can be seen from the results given in Table 7, the N-phenylcarbamates (I) of the invention show an excellent preventive effect on the drug-resistant strain but do not show any preventive effect on the tested drug-sensitive strain. On the contrary, commercially available known fungicides such as Benomyl and Thiophanate-methyl show a notable controlling effect on the drug-sensitive strain but not on the drug-resistant strain.

Experiment 5

Preventive effect on powdery mildew of cucumber (*Sphaerotheca fuliginea*):—

A plastic pot of 90 ml volume was filled with sandy soil, and seeds of cucumber (var: Sagami-hanjiro) were sowed therein. Cultivation was carried out in a greenhouse for 8 days. Onto the resulting seedlings having cotyledons, the test compound(s) formulated in emulsifiable concentrate or wettable powder and diluted with water were sprayed at a rate of 10 ml per pot. Then, the seedlings were inoculated with a mixed spore suspension of the drug-resistant and drug-sensitive strain of *Sphaerotheca fuliginea* by spraying and further cultivated in the greenhouse. Ten days thereafter, the infectious state of the plants were observed. The degree of damage was determined in the same manner as in Experiment 1, and the results are shown in Table 8.

Table 8

| Compound No. | Concentration of active ingredient (ppm) | Prevention value (%) |
|---|---|---|
| 14 | 100 | 34 |
| 14 | 20 | 0 |
| 18 | 100 | 42 |
| 18 | 20 | 0 |
| 22 | 100 | 36 |
| 22 | 20 | 0 |
| 23 | 100 | 44 |
| 23 | 20 | 0 |
| 36 | 100 | 36 |
| 36 | 20 | 0 |
| 43 | 100 | 41 |
| 43 | 20 | 0 |
| 45 | 100 | 34 |
| 45 | 20 | 0 |
| A | 100 | 45 |
| A | 20 | 12 |
| B | 500 | 42 |
| B | 100 | 10 |
| C | 100 | 42 |
| C | 20 | 8 |
| D | 500 | 36 |
| D | 100 | 0 |
| E | 100 | 44 |
| E | 20 | 10 |
| F | 100 | 43 |
| F | 20 | 8 |
| G | 100 | 42 |
| G | 20 | 8 |
| H | 100 | 40 |
| H | 20 | 5 |

(Continued)

| Compound No. | Concentration of active ingredient (ppm) | Prevention value (%) |
|---|---|---|
| 14 + A | 20 + 20 | 100 |
| 14 + B | 20 + 20 | 100 |
| 14 + E | 20 + 20 | 100 |
| 14 + G | 20 + 20 | 100 |
| 18 + C | 20 + 20 | 100 |
| 18 + D | 20 + 20 | 100 |
| 18 + F | 20 + 20 | 100 |
| 18 + H | 20 + 20 | 100 |
| 22 + A | 20 + 20 | 100 |
| 22 + D | 20 + 20 | 100 |
| 22 + E | 20 + 20 | 100 |
| 22 + G | 20 + 20 | 100 |
| 23 + A | 20 + 20 | 100 |
| 23 + B | 20 + 20 | 100 |
| 23 + E | 20 + 20 | 100 |
| 23 + G | 20 + 20 | 100 |
| 36 + A | 20 + 20 | 100 |
| 36 + B | 20 + 20 | 100 |
| 36 + E | 20 + 20 | 100 |
| 36 + G | 20 + 20 | 100 |
| 43 + C | 20 + 20 | 100 |
| 43 + D | 20 + 20 | 100 |
| 43 + F | 20 + 20 | 100 |
| 43 + H | 20 + 20 | 100 |
| 45 + A | 20 + 20 | 100 |
| 45 + D | 20 + 20 | 100 |
| 45 + E | 20 + 20 | 100 |
| 45 + G | 20 + 20 | 100 |

As can be seen from the results given in Table 8, the combined use of the N-phenylcarbamates (I) of the invention with benzimidazole thiophanate and/or cyclic imide fungicides show a better preventive effect than their sole use.

Experiment 6

Preventive effect on gray mold of tomato (*Botrytis cinerea*):—

A plastic pot of 90 ml volume was filled with sandy soil, and seeds of tomato (var: Fukuji No. 2) were sowed therein. Cultivation was carried out in a greenhouse for 4 weeks. Onto the resulting seedlings at the 4-leaf stage, the test compound(s) formulated in emulsifiable concentrate or wettable powder and diluted with water were sprayed at a rate of 10 ml per pot. Then, the seedlings were inoculated with a mixed spore suspension of the drug-resistant and drug-sensitive strain of *Botrytis cinerea* by spraying and placed at 20°C in a room of high humidity for 5 days. The degree of damage was determined in the same manner as in Experiment 1, and the results are shown in Table 9.

Table 9

| Compound No. | Concentration of active ingredient (ppm) | Prevention value (%) |
|---|---|---|
| 14 | 100 | 36 |
| 14 | 20 | 0 |
| 18 | 100 | 27 |
| 18 | 20 | 0 |
| 20 | 100 | 40 |
| 20 | 20 | 0 |
| 22 | 100 | 28 |
| 22 | 20 | 0 |
| 23 | 100 | 42 |
| 23 | 20 | 0 |
| 35 | 100 | 33 |
| 35 | 20 | 0 |
| 41 | 100 | 25 |
| 41 | 20 | 0 |
| 43 | 100 | 42 |
| 43 | 20 | 0 |
| 45 | 100 | 27 |
| 45 | 20 | 0 |
| I | 100 | 48 |
| I | 20 | 22 |
| J | 500 | 46 |
| J | 100 | 18 |
| K | 100 | 42 |
| K | 20 | 15 |
| L | 500 | 42 |
| L | 100 | 12 |

(Continued)

| Compound No. | Concentration of active ingredient (ppm) | Prevention value (%) |
|---|---|---|
| 14 + I | 20 + 50 | 100 |
| 14 + J | 20 + 50 | 100 |
| 18 + I | 20 + 50 | 100 |
| 18 + K | 20 + 50 | 100 |
| 20 + I | 20 + 50 | 100 |
| 20 + J | 20 + 50 | 100 |
| 20 + K | 20 + 50 | 100 |
| 20 + L | 20 + 50 | 100 |
| 22 + I | 20 + 50 | 100 |
| 22 + L | 20 + 50 | 100 |
| 23 + I | 20 + 50 | 100 |
| 23 + J | 20 + 50 | 100 |
| 23 + K | 20 + 50 | 100 |
| 23 + L | 20 + 50 | 100 |
| 35 + I | 20 + 50 | 100 |
| 35 + J | 20 + 50 | 100 |
| 41 + I | 20 + 50 | 100 |
| 41 + K | 20 + 50 | 100 |
| 43 + I | 20 + 50 | 100 |
| 43 + J | 20 + 50 | 100 |
| 43 + K | 20 + 50 | 100 |
| 43 + L | 20 + 50 | 100 |
| 45 + I | 20 + 50 | 100 |
| 45 + L | 20 + 50 | 100 |

As can be seen from the results given in Table 9, the combined use of the N-phenylcarbamates (I) of the invention with benzimidazole, thiophanate and/or cyclic imide fungicides show a better preventive effect than their sole use.

**Claims**

1. A fungicidal composition which comprises as an active ingredient a fungicidally effective amount of an N-phenylcarbamate of the formula:

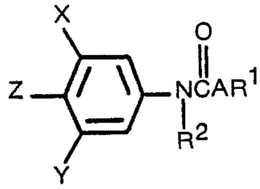

wherein

X is a chlorine atom, a methyl group or an ethynyl group;

Y is a methyl group, a $C_1$—$C_2$ alkoxy group or a methoxymethyl group;

Z is a $C_1$—$C_2$ alkoxy group, a $C_3$ alkenyloxy group, a $C_3$ alkynyloxy group or a halo $(C_1$—$C_2)$alkoxy group;

A is an oxygen atom or a sulfur atom;

$R^1$ is a $C_2$—$C_3$ alkyl group, a methoxy$(C_3)$alkyl group or a halo$(C_3)$alkyl group;

$R^2$ is a cyano group, a $C_1$—$C_2$ alkyl group, a $C_3$ alkenyl group, $C_3$ alkynyl group, a $C_1$—$C_2$ alkoxycarbonyl-$(C_1$—$C_2)$alkyl group or a cyclo$(C_3)$alkylmethyl group, or a group of the formula:

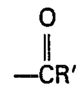

(in which R′ is a $C_1$—$C_2$ alkyl group, a cyclo$(C_3)$alkyl group, a phenoxymethyl group, a phenyl group or a phenylmethyl group), with the provisos that

when X is a chlorine atom, Y is not a methyl group;

when X is a chlorine atom, Y is $OC_2H_5$ and Z is $—OCH_2CH{=}CH_2$, A is not a sulfur atom;

when X is a chlorine atom and Y is a methoxymethyl group, $R^1$ is not a methoxy$(C_3)$alkyl group;

when X is a chlorine atom and Y is a methoxymethyl group, $R^2$ is not a $C_1$—$C_2$ alkoxycarbonylmethyl group; and

when X is an ethynyl group and Y is a $C_1$—$C_2$ alkoxy group, $R^2$ is not a $C_1$—$C_3$ alkyl group.

2. A fungicidal composition as claimed in claim 1 which further comprises as an additional active ingredient(s) a benzimidazole, thiophanate and/or a cyclic imide fungicide.

3. A fungicidal composition as claimed in claim 2 wherein the benzimidazole fungicide is methyl 1-(butylcarbamoyl)benzimidazol-2-ylcarbamate, 2-(2-furyl)benzimidazole, 2-(4-thiazolyl)benzimidazole or methyl benzimidazol-2-ylcarbamate.

4. A fungicidal composition as claimed in claim 2 wherein the thiophanate fungicide is 1,2-bis(3-methoxycarbonyl-2-thioureido)benzene, 1,2-bis(3-ethoxycarbonyl-2-thioureido)-benzene, 2-(O,S-dimethylphosphorylamino)-1-(3′-methoxycarbonyl-2′-thioureido)benzene or 2-(O,O-dimethylthiophosphorylamino)-1-(3′-methoxycarbonyl-2′-thioureido)benzene.

5. A fungicidal composition as claimed in claim 2 wherein the cyclic imide fungicide is 3-(3′,5′-dichlorophenyl)-1,2-dimethylcyclopropane-1,2-dicarboximide, 3-(3′,5′-dichlorophenyl)-1-isopropylcarbamoylimidazolidine-2,4-dione, 3-(3′,5′-dichlorophenyl)methyl-5-vinyloxazolidine-2,4-dione or ethyl-(RS)-3-(3′,5′ dichlorophenyl)-5-methyl-2,4-dioxo-oxazolidine-5-carboxylate.

6. An N-phenylcarbamate of the formula:

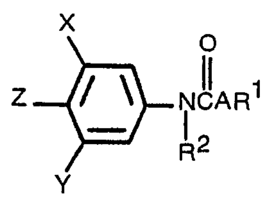

wherein X, Y, Z, A, $R^1$ and $R^2$ are each as defined in claim 1.

7. A method for controlling plant pathogenic fungi which comprises applying a fungicidally effective amount of at least one N-phenylcarbamate of the formula:

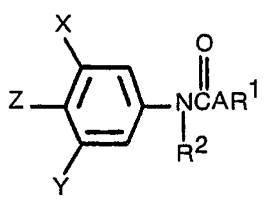

wherein X, Y, Z, A, $R^1$ and $R^2$ are each as defined in claim 1, to plant pathogenic fungi.

8. A method as claimed in claim 7, wherein the plant pathogenic fungi is resistant to benzimidazole, thiophanate and/or cyclic imide fungicides.

9. A method for controlling plant pathogenic fungi which comprises applying a fungicidally effective amount of a mixture of the N-phenylcarbamate of the formula:

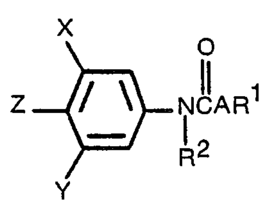

wherein X, Y, Z, A, $R^1$ and $R^2$ are each as defined in claim 1 and a benzimidazole, thiophanate and/or a cyclic imide fungicide, to plant pathogenic fungi.

10. A process for producing an N-phenylcarbamate of the formula:

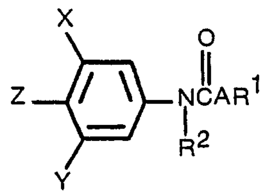

wherein X, Y, Z, A, $R^1$ and $R^2$ are as defined in claim 1 which process comprises (a) reacting an N-phenylcarbamate of the formula:

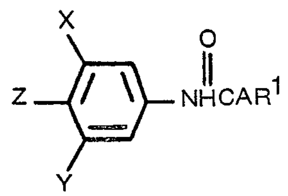

wherein X, Y, Z, A and $R^1$ are each as defined above, with a halide or an acid anhydride of the formula:

$R^2$—W or $(R'CO)_2O$

wherein $R^2$ and $R^1$ are as defined in claim 1 and W is halogen; or (b) reacting an aniline derivative of the formula:

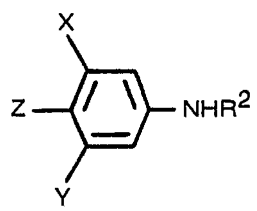 (V)

wherein X, Y, Z and $R^2$ are each as defined above, with a chloroformate of the formula:

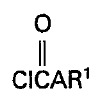

wherein A and $R^1$ are each as defined above.

**Patentansprüche**

1. Fungizid, enthaltend als wirksamen Bestandteil eine fungizid wirksame Menge eines N-Phenylcarbamats der Formel:

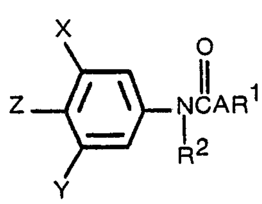

in der

X ein Chloratom, eine Methyl- oder eine Äthinylgruppe bedeutet;

Y eine Methylgruppe, einen $C_1$—$C_2$-Alkoxyrest oder eine Methoxymethylgruppe darstellt;

Z einen $C_1$—$C_2$-Alkoxyrest, einen $C_3$-Alkenyloxyrest, einen $C_3$-Alkinyloxyrest oder einen Halogen-($C_1$—$C_2$)-alkoxyrest bedeutet;

A ein Sauerstoffatom oder ein Schwefelatom ist;

$R^1$ einen $C_2$—$C_3$-Alkylrest, einen Methoxy-($C_3$)-alkylrest oder einen Halogen-($C_3$)-alkylrest bedeutet;

$R^2$ eine Cyanogruppe, einen $C_1$—$C_2$-Alkylrest, einen $C_3$-Alkenylrest, einen $C_3$-Alkinylrest, einen $C_1$—$C_2$-Alkoxycarbonyl-($C_1$—$C_2$)-alkylrest oder eine Cyclo-($C_3$)-alkylmethylgruppe, oder einen Rest der Formel

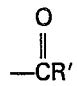

bedeutet (wobei R′ einen $C_1$—$C_2$-Alkylrest, einen Cyclo($C_3$)-alkylrest, eine Phenoxymethylgruppe, eine Phenylgruppe oder eine Phenylmethylgruppe darstellt), mit den Maßgaben, daß

falls X ein Chloratom ist, Y keine Methylgruppe darstellt;

falls X ein Chloratom ist, Y den Rest $OC_2H_5$ und Z den Rest $—OCH_2CH{=}CH_2$ bedeutet, A kein Schwefelatom darstellt;

falls X ein Chloratom ist und Y eine Methoxymethylgruppe ist, $R^1$ keinen Methoxy($C_3$)-alkylrest bedeutet;

falls X ein Chloratom und Y eine Methoxyäthylgruppe bedeuten, $R^2$ keinen $C_1$—$C_2$-Alkoxycarbonylmethylrest darstellt; und

falls X ein Äthinylrest und Y einen $C_1$—$C_2$-Alkoxyrest darstellt, $R^2$ keinen $C_1$—$C_3$-Alkylrest darstellt.

2. Fungizid nach Anspruch 1, das außerdem als zusätzliche(n) aktive(n) Bestandteil(e) ein Benzimidazol-, Thiophanat- und/oder cyclisches Imid-Fungizid enthält.

3. Fungizid nach Anspruch 2, wobei das Benzimidazol-Fungizid Methyl-1-(butylcarbamoyl)-benzimidazol-2-ylcarbamat, 2-(2-Furyl)-benzimidazol, 2-(4-Thiazolyl)-benzimidazol oder Methylbenzimidazol-2-ylcarbamat ist.

4. Fungizid nach Anspruch 2, wobei das Thiophanat-Fungizid 1,2-Bis(3-methoxycarbonyl-2-thioureido)-benzol, 1,2-Bis-(3-äthoxycarbonyl-2-thioureido)-benzol, 2-(O,S-Dimethylphosphorylamino)-1-(3′-methoxycarbonyl-2′-thioureido)-benzol oder 2-(O,O-Dimethylthiophosphorylamino)-1-(3′-methoxycarbonyl-2′-thioureido)-benzol ist.

5. Fungizid nach Anspruch 2, wobei das cyclische Imid-Fungizid 3-(3′5′-Dichlorphenyl)-1,2-dimethylcyclopropan-1,2-dicarboximid, 3-(3′,5′-Dichlorphenyl)-1-isopropylcarbamoylimidazolidon-2,4-dion, 3-(3′,5′-Dichlorphenyl)-methyl-5-vinyloxazolidin-2,4-dion oder Äthyl-(R,S)-3-(3′,5′-dichlorphenyl)-5-methyl-2,4-dioxooxazolidin-5-carboxylat ist.

6. N-Phenylcarbamat der Formel:

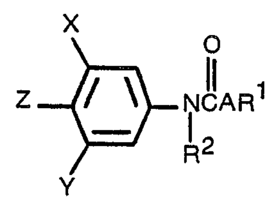

in der X, Y, Z, A, $R^1$ und $R^2$ die in Anspruch 1 angegebene Bedeutung haben.

7. Verfahren zur Bekämpfung von pflanzenpathogenen Pilzen, das Aufbringen einer fungizid wirksamen Menge mindestens eines N-Phenylcarbamates der Formel:

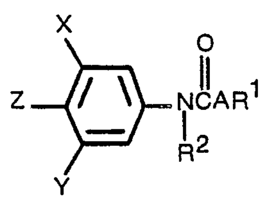

in der X, Y, Z, A, $R^1$ und $R^2$ die in Anspruch 1 angegebene Bedeutung haben, auf die pflanzenpathogenen Pilze umfaßt.

8. Verfahren nach Anspruch 7, wobei die pflanzenpathogenen Pilze gegen Benzimidazol-, Thiophanat- und/oder cyclische Imid-Fungizide resistent sind.

9. Verfahren zum Bekämpfen von pflanzenpathogenen Pilzen, das Aufbringen einer fungizid wirksamen Menge eines Gemisches aus dem N-Phenylcarbamat der Formel:

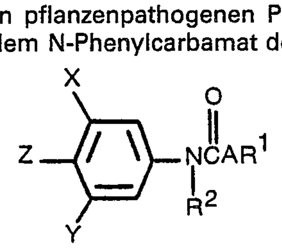

in der X, Y, Z, A, $R^1$ und $R^2$ die in Anspruch 1 angegebene Bedeutung haben, und einem Benzimidazol-, Thiophanat- und/oder einem cyclischen Imid-Fungizid, auf die pflanzenpathogenen Pilze umfaßt.

10. Verfahren zur Herstellung eines N-Phenylcarbamates der Formel:

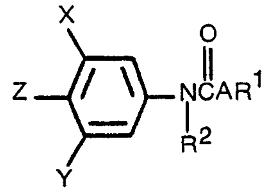

in der X, Y, Z, A, $R^1$ und $R^2$ die in Anspruch 1 angegebene Bedeutung haben, das (a) Umsetzung eines N-Phenylcarbamates der Formel:

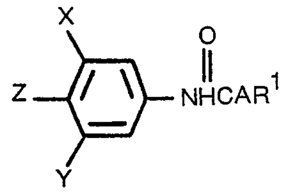

in der X, Y, Z, A und $R^1$ die vorstehend genannte Bedeutung haben, mit einem Halogenid oder einem Säureanhydrid der Formel

$R^2$—W oder $(R'CO)_2O$

in denen $R^2$ und $R^1$ die in Anspruch 1 angegebenen Bedeutung haben und W ein Halogenatom ist; oder (b) Umsetzung eines Anilinderivats der Formel:

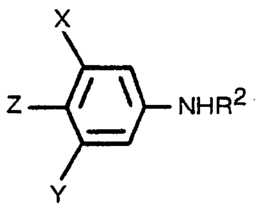

(V)

in der X, Y, Z und $R^2$ die vorstehend genannte Bedeutung haben, mit einem Chlorameisensäureester der Formel:

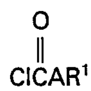

in der A und $R^1$ die vorstehend genannte Bedeutung haben, umfaßt.

**Revendications**

1. Composition fongicide, caractérisée en ce qu'elle comprend, à titre d'ingrédient actif, une quantité efficace du point de vue fongicide d'un N-phénylcarbamate répondant à la formule qui suit:

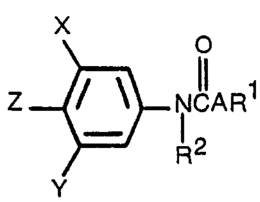

dans laquelle

X représente un atome de chlore, un radical méthyle ou un radical éthynyle,

Y représente un radical méthyle, un radical alcoxy en $C_1$—$C_2$ ou un radical méthoxyméthyle;

Z représente un radical alcoxy en $C_1$—$C_2$, un radical alcényloxy en $C_3$, un radical alcynyloxy en $C_3$, ou un radical haloalcoxy en $C_1$—$C_2$;

A représente un atome d'oxygène ou atome de soufre, $R^1$ représente un radical alkyle en $C_2$—$C_3$, un radical méthoxyalkyle($C_3$), ou un radical haloalkyle en $C_3$;

$R^2$ représente un radical cyano, un radical alkyle en $C_1$—$C_2$, un radical alcényle en $C_3$, un radical

alcynyle en $C_3$, un radical alcoxy($C_1$—$C_2$)carbonyl($C_1$—$C_2$)alkyle ou un radical cycloalkyl($C_3$)méthyle, ou un radical de la formule

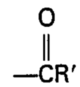

(dans laquelle R′ représente un radical alkyle en $C_1$—$C_2$, un radical cycloalkyle en $C_3$, un radical phénoxyméthyle, un radical phényle ou un radical phénylméthyle), avec les conditions que

lorsque X représente un atome de chlore, Y ne représente pas de radical méthyle;

lorsque X représente un atome de chlore, Y soit $OC_2H_5$ et Z soit $—OCH_2CH{=}CH_2$, A ne représente pas d'atome de soufre;

lorsque X est un atome de chlore et Y est un radical méthoxyméthyle, $R^1$ ne représente pas de radical méthoxyalkyle($C_3$);

lorsque X est un atome de chlore et Y est un radical méthoxyméthyle, $R^2$ ne soit pas de radical alcoxy($C_1$—$C_2$)carbonylméthyle; et

lorsque X représente un radical éthynyle et Y représente un radical alcoxy en $C_1$—$C_2$, $R^2$ ne soit pas de radical alkyle en $C_1$—$C_3$.

2. Composition fongicide suivant la revendication 1, caractérisée en ce qu'elle comprend, à titre d'un ingrédient ou d'ingrédients actifs supplémentaires, au moins un fongicide du type benzimidazole, thiophanate et/ou imide cyclique.

3. Composition fongicide suivant la revendication 2, caractérisée en ce que le fongicide du type benzimidazole est le 1-(butylcarbamoyl)benzimidazole-2-ylcarbamate de méthyle, le 2-(2-furyl)benzimidazole, le 2-(4-thiazolyl)benzimidazole ou le benzimidazole-2-ylcarbamate de méthyle.

4. Composition fongicide suivant la revendication 2, caractérisée en ce que le fongicide du type thiophanate est le 1,2-bis(3-méthoxycarbonyl-2-thiouréido)benzène, le 1,2-bis(3-éthoxycarbonyl-2-thiouréido)benzène, le 2-(O,S-diméthylphosphorylamino)-1-(3′-méthoxycarbonyl-2′-thiouréido)benzène ou le 2-(O,O-diméthylthiophosphorylamino)-1-(3′-méthoxycarbonyl-2′-thiouréido)benzène.

5. Composition fongicide suivant la revendication 2, caractérisée en ce que le fongicide du type imide cyclique est le 3-(3′,5′-dichlorophényl)-1,2-diméthylcyclopropane-1,2-dicarboximide, la 3-(3′,5′-dichlorophényl)-1-isopropylcarbamoylimidazolidine-2,4-dione, la 3-(3′,5′-dichlorophényl)méthyl-5-vinyloxazolidine-2,4-dione ou le (RS)-3-(3′,5′-dichlorophényl)-5-méthyl-2,4-dioxooxazolidine-5-carboxylate d'éthyle.

6. N-Phénylcarbamates de la formule:

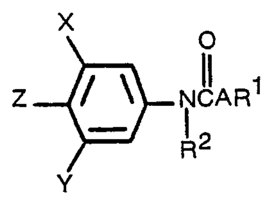

dans laquelle X, Y, Z, A, $R^1$ et $R^2$ possèdent chacun les significations qui leur ont été attribuées dans la revendication 1.

7. Procédé pour combattre des champignons phytopathogènes, caractérisé en ce que l'on applique une quantité efficace du point de vue fongicide d'au moins un N-phénylcarbamate de la formule:

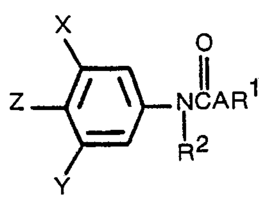

dans laquelle X, Y, Z, A, $R^1$ et $R^2$ possèdent chacun les significations qui leur ont été attribuées dans la revendication 1, à des champignons phytopathogènes.

8. Procédé suivant la revendication 7, caractérisé en ce que le champignon phytopathogène est résistant aux fongicides du type benzimidazole, thiophanate et/ou imide cyclique.

9. Procédé pour combattre des champignons phytopathogènes, caractérisé en ce que l'on applique une quantité efficace du point de vue fongicide d'un mélange d'un N-phénylcarbamate de la formule:

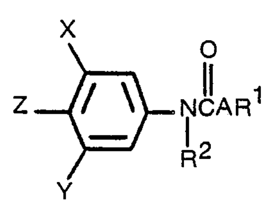

dans laquelle X, Y, Z, A, $R^1$ et $R^2$ possèdent chacun les significations qui leur ont été attribuées dans la revendication 1 et un fongicide du type benzimidazole, thiophanate et/ou imide cyclique, à des champignons phytopathogènes.

10. Procédé de fabrication d'un N-phénylcarbamate de la formule:

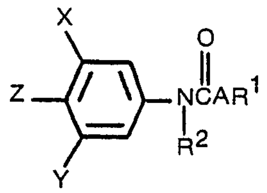

dans laquelle X, Y, Z, A, $R^1$ et $R^2$ possèdent chacun les significations qui leur ont été attribuées dans la revendication 1, caractérisé en ce que (a) on fait réagir un N-phénylcarbamate de la formule:

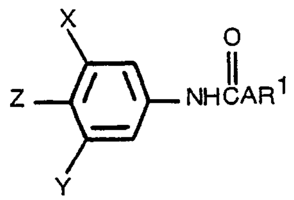

(II)

dans laquelle X, Y, Z, A et $R^1$ possèdent chacun les significations qui leur ont été attribuées ci-dessus, sur un halogénure ou un anhydride d'acide de la formule

$R^2$—W ou $(R'CO)_2O$

dans lesquelles $R^1$ et $R^2$ possèdent chacun les significations qui leur ont été attribuées dans la revendication 1 et W représente un atome d'halogène; ou bien (b) on fait réagir un dérivé de l'aniline de la formule:

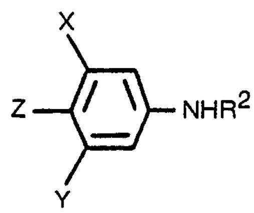

(V)

dans laquelle X, Y, Z et $R^2$ possèdent chacun les significations qui leur ont été attribuées ci-dessus, sur un chloroformiate de la formule:

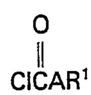

dans laquelle A et $R^1$ possèdent chacun les significations qui leur ont été attribuées ci-dessus.